# EUROPEAN PATENT SPECIFICATION

(11) **EP 0 833 585 B1**
(45) Date of publication and mention of the grant of the patent: **11.08.1999**
(21) Application number: 96920947.7
(22) Date of filing: 21.06.1996
(51) Int. Cl.: A61B 5/05

(54) **METHOD AND APPARATUS FOR USE IN IMAGING A BODY**
VERFAHREN UND VORRICHTUNG ZUR ABBILDUNG VON KÖRPERN
PROCEDE ET APPAREIL UTILES DANS DES APPLICATIONS D'IMAGERIE MEDICALE

(30) Priority: 22.06.1995 GB 9512717
(43) Date of publication of application: 08.04.1998
(73) Proprietor: BTG INTERNATIONAL LIMITED, London EC4M 7SB (GB)
(72) Inventor: BOONE, Kevin, Graham, London N1 7BJ (GB); HOLDER, David, Simon, London N7 0AH (GB)
(74) Representative: Bird, Christopher John
(86) International application number: GB9601499
(87) International publication number: WO9700642

(56) References cited:
- WO-A-88/07392
- WO-A-91/19454
- DE-A- 4 301 483
- MEDICAL AND BIOLOGICAL ENGINEERING AND COMPUTING, vol. 30, no. 2, STEVENAGE GB, pages 140-146, XP000271685 D.S. HOLDER: "Impedance changes during the compound nerve action potential: implications for impedance imaging of neuronal depolarisation in the brain"
- PROC. OF ANNUAL INTERNATIONAL CONF. OF IEEE ENGINEERING IN MEDICINE & BIOLOGY SOCIETY, vol. 15, 28 - 31 October 1993, SAN DIEGO (US), pages 86-87, XP000436668 R. GUARDO ET AL.: "A superheterodyne serial data acquisition system for Electrical Impedance Tomography"

## Description

The present invention relates to imaging, and more particularly to a method and apparatus for use in imaging a body by means of the technique of electrical impedance tomography (EIT), also termed "applied potential tomography" (APT). In particular, the invention may be used in imaging neurological function within the body.

Over the past few years there has been considerable interest in clinical applications of EIT, and such applications have extended to areas such as imaging changes in the thorax during breathing, in the stomach during gastric emptying and in the heart during intraventricular haemorrhage. EIT involves the application of spaced electrodes to the skin surface of a body under investigation, usually in the form of a belt around the body such that the electrodes lie in the plane of the body to be investigated. In a typical biomedical EIT system, low voltage alternating electrical current is applied between two neighbouring electrodes (known as a drive pair) and the resulting potentials measured between pairs (known as driven pairs) of all the remaining electrodes. The potentials are recorded and the sequence repeated for all the other drive pairs, giving a matrix of measured impedance signal values. For example, the use of 16 electrodes provides a total of 104 independent impedance measurements. Using known reconstruction techniques this matrix can be processed by appropriate computer hardware to regenerate an image of impedance within the body plane. EIT methods and equipment have undergone considerable development and by making use of techniques such as parallel data collection and noise reduction, real time systems are now available capable of providing clinically useful images of dynamic phenomena. The basis of EIT is described in more detail in the paper "Applied Potential Tomography", Barber & Brown, 1984, J. Phys. E: Sci. Instrum.,17, 723-733. It is to be noted that neither the drive pair nor the receive pair need to comprise two adjacent electrodes, and in some cases a diametric drive pair and/or receive pair may offer certain advantages for clinical measurements.

One potentially interesting area for application of EIT techniques is in the clinical neurosciences, where scalp measurements are used to obtain images of functional changes in the human or animal brain. It is possible with existing equipment to form images of neural functional activity, such as that associated with stroke or spreading depression in a patient, due to the fact that cell swelling within the cortex of such subjects produces impedance increases of up to 30% ("Electrical impedance tomography with cortical or scalp electrodes during global cerebral ischaemia in the anaesthetised rat", D S Holder, Clin. Phys. Physiol. Meas. 1989, Vol. 13, No. 1, 87-98, and "Imaging of cortical spreading depression by EIT: Implications for localization of epileptic foci", K Boone, A M Lewis and D S Holder, Physiol. Meas. 15 (1994) A189-A198).

It has also been suggested that the much smaller changes in impedance during neuronal discharge might be measured by EIT techniques. ("Impedance changes during evoked nervous activity in human subjects: implications for the application of applied potential tomography (APT) to imaging neuronal discharge" D.S. Holder, Clin. Phys. Physiol. Meas. 1989, Vol. 10, No. 3, 267-274). The principle of this application is that the impedance of the neuronal membranes is known to fall during the action potential or during the sub-threshold depolarisations which accompany synaptic activity, and there may be other related effects such as the movement of ions between intra- and extra-cellular compartments. By use of scalp electrodes, changes in impedance taking place in the brain may be recorded and used to image the progress of information along circuits within the brain. For example, the brain may be stimulated by a visual signal and EIT images subsequently reconstructed for each millisecond or so of the recording window, thus enabling the resultant action potential processes to be tracked along their pathways in the subject's brain. There is no established technique which at present permits accurate imaging of neuronal depolarisation with millisecond or sub-millisecond time resolution. MRI and PET techniques produce images of cerebral activity, but these are related to metabolic recovery processes, which occur over seconds or minutes.

One of the problems with this approach is that the impedance changes associated with action potentials are generally very small and very rapid. Even if there is a substantial decrease in the resistance of the neuronal membranes themselves when they depolarise, the impedance of the tissue as a whole may not change in the same proportion, because the amount of current that enters the neuronal axoplasm depends more on the absolute impedance of the nerve axons and their geometry than it does on the membrane impedance. One study has estimated that the changes in impedance are, at most, between 0.1 and 1% of the resting impedance, depending on the area of the brain concerned. "Impedance changes during the compound nerve action potential: implications for impedance imaging of neuronal depolarisation in the brain", D.S. Holder, Med. & Biol. Eng. & Comp. (March 1992), Vol. 30, No. 2, 140-146 describes research verifying the definite but low level of impedance change measurable in isolated crab nerves. It is clear that the resolution and sensitivity of an EIT system will need to be very high to accurately image such small changes using measurements taken using scalp electrodes, and currently available devices are not capable of producing such images.

Investigations into the above-mentioned technique of imaging action potentials have been carried out using a prototype system employing square wave excitation of 50 µA at 5 Hz applied to a saline phantom. The square wave signal was chosen because the capacitive properties of the cell membranes mean that the high frequencies used in conventional EIT systems are generally unsuitable. The resulting inter-electrode potentials were sampled at a rate of 4000 frames per second and 100 sets of frames were averaged to produce the results. The results of the experiment showed a signal-to-noise ratio of 40-50 dB and reciprocity errors of 10% - 20%. Images of discrete resistivity changes of less than 10% could be obtained but with significant systematic errors. The prototype was thus found to be unsuitable for neurophysiological imaging.

One of the problems of the prototype system has been that of noise. With the square Wave signal used the amplifiers will detect a higher level of extraneous noise than would be the case with an AC system. 50 Hz mains interference lies within the recording bandwidth, as does intrinsic EEG activity associated with the action potentials. Previous systems have attempted to minimise the noise effects by filtering or averaging, but such attempts have met with limited success.

It is an object of the present invention to provide an improved system suitable for use in EIT. According to a first aspect of the invention, there is provided a method for use in imaging a body by means of the technique of EIT comprising the steps of providing a plurality of electrodes in electrical contact with the body around the periphery of the body. applying a first electrical input signal to at least one of the electrodes over a first time period applying a second electrical input signal to the at least one of the electrodes over a subsequent second time period, the second electrical input signal being an inverted form of the first electrical input signal, measuring the resulting electrical output signal at one or more of the remaining electrodes over the first and second time periods, and calculating the difference between the measured signal obtained during the first time period and that obtained during the second time period, to provide a difference signal.

Preferably, the first and second electrical input signals are unidirectional signals of equal amplitude and respectively opposite sign.

In a preferred form, the applications of the first electrical input signal and that of the second electrical input signal are continuously alternated, the first and second time periods being equal, and the electrical signal measurements are taken at regular intervals during each time period.

The measurement step of the invention is preferably carried out on a number of different pluralities of electrodes, and images representative of the body are generated using said difference signal.

The signal application step may be carried out in synchronisation with the application of a separate stimulus signal to the body.

In optimising the method of the invention, it has been determined that the first and second time period are preferably each between 0.1 and 1.0 seconds.

According to a second aspect of the invention, there is provided apparatus for use in imaging a body by means of the technique of EIT comprising a plurality of electrodes adapted for electrical contact with the body around the periphery of the body, means for applying a first electrical input signal to at least one of the electrodes over a first time period, means for providing an inverted form of the first electrical input signal to produce a second electrical input signal, means for applying the second electrical input signal to the at least one of the electrodes over a second, subsequent time period, means for measuring the resulting electrical output signals at one or more of the remaining electrodes over the first and second time periods, and means for calculating the difference between the measured signal obtained during the first time period and that obtained during the second time period, to provide a difference signal.

The means for providing an inverted form of the first electrical signal to produce the second electrical signal may comprise a switching means controlling an electrical current generator, electrically isolated from the measuring means. Said means may comprise one or more optical isolators.

The invention will now be described in more detail by way of example with reference to the accompanying figures, in which:
Figure 1 shows in schematic form the main components of a 16 electrode EIT system as applied to neuronal imaging of a human subject;
Figure 2 illustrates an applied form of current excitation along with applied external stimulus pulses;
Figure 3 schematically represents an example of measured potential difference (V) due to various sources, shown on a common axis;
Figure 4 is a circuit diagram of an isolated current generator; and
Figure 5 illustrates a sequence of successive data acquisition cycles used in tests.

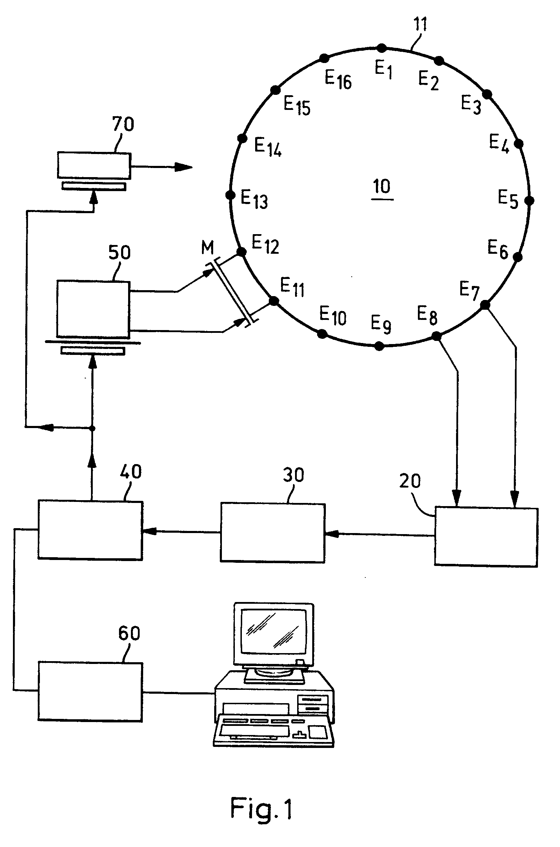

Scalp electrodes E₁ to E₁₆ are shown in contact with the surface of subject's head 10 in Figure 1. The electrodes are equally spaced and for this purpose may be provided on a flexible band 11 for selective positioning and attachment. Each electrode is connected by a lead to a switching means, such as a computer controlled current multiplexer M, whereby electrodes may be selected for application of a fixed current according to a desired excitation sequence. This general concept is well known in EIT techniques in general and will not be further described here. Figure 1 shows an electrical signal from current generator 50 being applied between adjacent electrodes E₁₁ and E₁₂, whilst reference 70 represents a stimulus pulse generator (see below).

Signal measurements, in this case potential difference measurements, are made between others of the electrodes, and again methods of carrying out such measurements are generally known in the field of conventional EIT. It is noted that signal measurements made between different groups (e.g. pairs) of electrodes may be made sequentially or simultaneously (the latter technique known as parallel data collection). In the system illustrated in Figure 1, signal measurement is carried out in parallel using 16 instrumentation amplifiers, each connected between an adjacent pair of electrodes. The figure illustrates only the measurement of the potential difference between electrodes E₇ and E₈ by way of amplifier 20.

The amplified measured electrical signals are then amplified and passed via signal processing means, such as a 16-bit analogue-to-digital converter 30 and a digital interface board 40, to be processed into an image by reconstruction software. Again, various reconstruction techniques and specific algorithms are known within the general field of EIT and will not be further described here. The resulting images represent tomographic images of the distribution of electrical characteristics across the cross section of the subject's brain in which the electrodes lie. The data acquisition control, image reconstruction, display and recording are all carried out by a microcomputer 60, which also provides a signal to drive a stimulus pulse device 70 (see below).

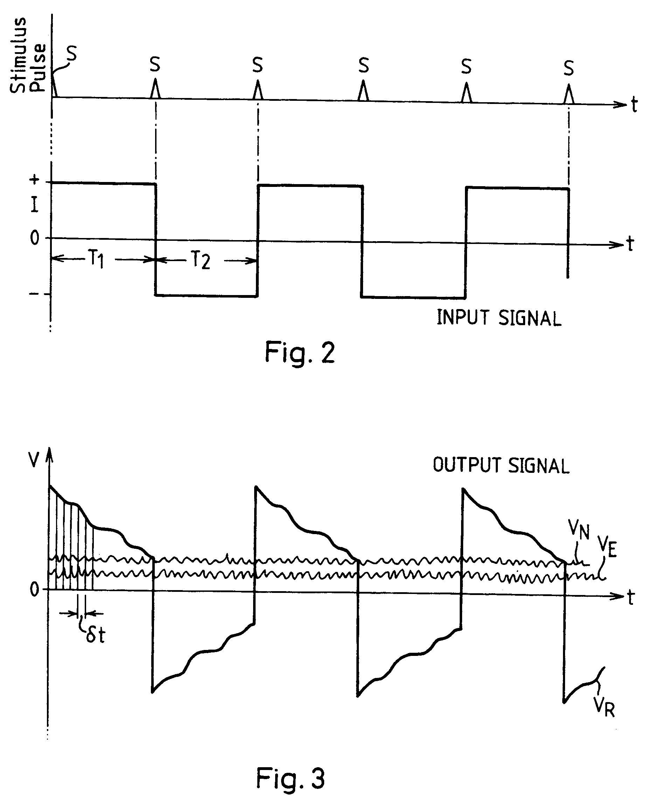

The lower trace of Figure 2 illustrates the applied current excitation. As stated above, AC excitation is not well suited to investigating the activity of cells within the brain due to their capacitive character. According to the invention a bipolar waveform is selected, successively reversing the direction of constant current from a time period T₁ in which the signal is positive, to a time period T₂ in which the signals is of equal amplitude but of opposite, negative, polarity. An appropriate time for both periods T₁ and T₂ is 100 ms, and an appropriate current for neurophysiological application is 50µA.

Figure 2 also shows successive stimulus pulses S from generator 70, and in this case the pulses are coincident with the commencement of each period of the input current wave (upper trace). These stimulus pulses represent an external stimulation of the neurological function such as a flash of light (flash visual evoked response) or electrical stimulation of a nerve such as the median nerve at the wrist (sensory evoked response).

As Figure 3 illustrates, the signal is measured at a prescribed sampling rate with a time interval between samples of δt, representing preferably a large number of samples per time period of signal application. The signal measured can be seen as the sum of three components, namely that due to the dynamic impedance change taking place, V_{R}, that due to the patient's EEG signal, V_{E}, and that due to background noise such as the 50 Hz mains component, V_{N}. The V_{E} component includes an electrical signal component evoked by the stimulus signals, ie. due to the action potential itself. It will be appreciated that the figure is illustrative only and not to scale, being purely for purposes of explanation of the invention. As the figure shows, V_{R} follows the polarity of the applied signal, whilst the other components are largely independent of the applied signal. The unwanted noise components V_{N} and V_{E} can be cancelled or at least significantly reduced by subtracting signals measured during time period T₂ from those measured at corresponding points during time period T₁. Components V_{R} will reinforce, whilst those components independent of the bipolar input signal (ie. V_{N}, V_{E}) will effectively cancel. The technique therefore makes use of all the measured data, the subsequent image reconstruction then being carried out using the signal representing the subtracted signals. Although the excitation polarity is reversed between each measurement time period, the biological response from nerve (2-10ms) is very much shorter than this, and the excitation can therefore be considered equivalent to DC.

In practice, to enhance the signal-to-noise ratio, the method is carried out by averaging measured signals over a succession of repeated stimulus pulses, the collected averaged data then being used for image reconstruction. Data are digitised and stored on the microcomputer 60.

### Design and Calibration

The impedance measuring system described below was developed in respect of tests carried out firstly on a prepared crab nerve (in which resistivity changes are known to be up to 2.5%) using a tetrapolar arrangement and using an isolated constant voltage stimulator in electrical contact with the nerve as a synchronised stimulus, and secondly on the cerebral cortex of two anaesthetised rabbits, the resistance changes being evoked by a stimulus to the median nerve, again by way of an isolated constant voltage source.

For the crab nerve tests, the nerve was isolated in each case and suspended from the set of electrodes. The voltage stimulus was applied at one end of the nerve to generate an action potential, which propagated past the electrodes. For the rabbit cortex tests, a tetrapolar arrangement was again selected, transcranial electrodes applied over the most active part of the cortex. For both sets of test, an excitation current of about 10µA was selected.

From these single channel measurements made on animal tissue the *in-vivo* measurements of the DC impedance showed clear changes during depolarisation. In the case of the crab nerve, with an electrode spacing of 1mm, the impedance measured along the length of the fibres was found to decrease during depolarisation by 0.2-2.5% (mean value 1.0%). The resistance change measured perpendicular to the direction of the fibres was about 0.01%. In the case of the sensory cortex of the rabbit, these changes were found to be small but detectable, of the order of 0.01-0.03%.

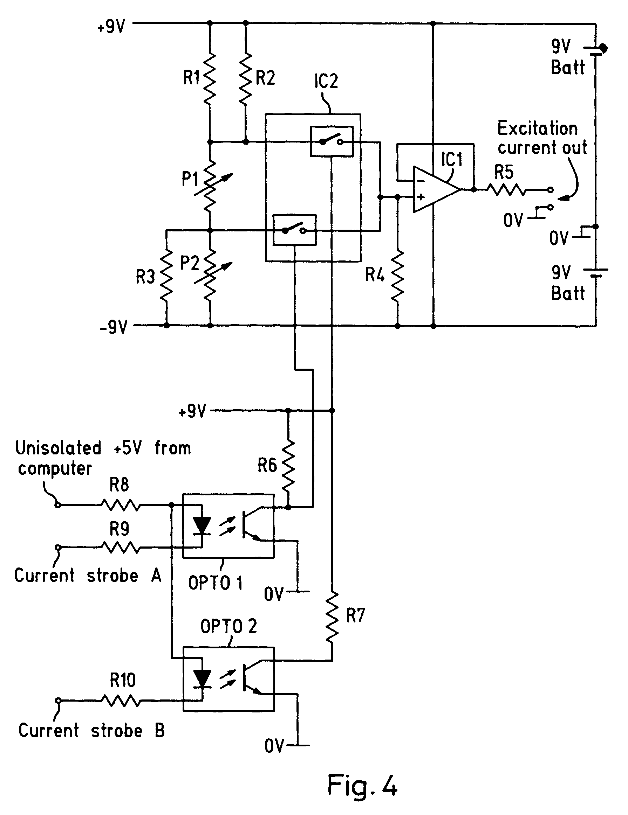

The excitation protocol (that is, the reversal of polarity of excitation signal), has the advantage that as well as cancelling as far as possible unwanted signal components, it also serves to limit the extent of electrode polarisation and to reduce any potential risk to the tissue under study. The impedance of the bare metal electrodes was found to be 2-10kΩ even with this excitation protocol. To avoid the generation of common-mode voltages by the current source a current generator 50 (Figure 1) electrically isolated from the recording system is used. The isolated current generator provides a current whose polarity is selected by the microcomputer 60 via a digital interface board 40 (see Figure 1). A circuit diagram of the isolated current generator is shown in Figure 4, and Table 1 gives values and specifications of the various components of the circuit.

Isolation is achieved by way of optical isolators OPTO 1 and OPTO2 (Siemens SFH618-3-X001). Such a device consists essentially of an LED and a photo-transistor and provides a very high isolation voltage (>5000V). The computer selects the current polarity by switching using 'Current Strobe A' or 'Current Strobe B'. The term 'current strobe' is used herein to indicate a device producing a control signal which is able to cause selection of alternative conditions, in this instance, of alternative current configurations.

The constant current is achieved by connecting a constant voltage from the output of IC1 to the electrodes, via a large resistance R5 (500KΩ). The size of the voltage is determined by switching equal and opposite control voltages from two 9V batteries connected in series onto the non-inverting terminal of IC1 using the analogue switches in IC2. The control voltages are derived from the resistor network consisting of resistances R1, R2 and R3 and trimmers P1 and P2. It is desirable to reduce as far as possible the stimulation of the nervous tissue under study by the current excitation itself. A current below the threshold for nerve stimulation is therefore preferably employed, and the circuit was designed such that adjustment of P1 sets the overall current level form 0-12µA as follows. With P1 at its maximum value, the control voltage at the input of IC1 approaches the positive or negative supply voltage, depending on the settings of Current Strobes A and B. With P1 at its minimum value, the control voltage is close to zero.

**Table 1**

| Component | Value |
|---|---|
| R1, 3 | 1k |
| R2 | 27k |
| R4 | 120k |
| R5 | 500k |
| R6, 7 | 2k2 |
| R8, 9, 10 | 150R |
| P1 | 100k |
| P2 | 50k |
| IC1 | LF351 |
| IC2 | DG201 |
| OPTO1, 2 | SFH618-3-X001 |

For the control voltages to be equal and opposite, the resistances of parallel combinations R1|R2 and R3|P2 must be equal, as must the two battery voltages. In practice the batteries do not generate exactly equal voltages, and P2 was adjusted to balance the positive and negative output currents. Because the resistance of P2 is much higher than that of R3, P2 provides a very fine adjustment of the parallel resistance R3|P2. Re-balancing is necessary whenever either battery is replaced. It is to be noted that the ground points in the isolated current generator circuit refer to the junction of the two 9V batteries ('0V' in Figure 4). It is important that this point remains internal to the generator, to avoid any current path between the generator and the computer or the differential amplifiers.

The differential amplifier 20 is of a known design using two different split supply rails set at voltages found to provide the best common-mode rejection. The gain is normally ×100, although ×10 and ×1000 were found to be occasionally useful in trials. Passive first-order filtering circuits were incorporated, serving to prevent any time-domain ripple that might manifest itself as a biological signal.

The data acquisition cycle is controlled by a computer programme, and in the tests a programme written in C++ for the Microsoft Windows environment on a Pentium(TM)-based PC was used. The maximum sampling rate possible was found to be 6000 samples per second, which was used for all measurements.

In practical application to EIT, the current multiplexer selecting electrodes for input signal application is controlled by the same programme, allowing any combination of drive electrodes to be selected, and voltage measurement is carried out using 16 instrumentation amplifiers, each connected between a adjacent pair of electrodes around the body under investigation.

The programme produced a record of comparison of the signals resulting from successive current applications of opposite polarity by subtracting the measurements of alternate cycles. The first two data acquisition cycles were as follows:
1. Set current strobe A high to switch on current generator; polarity positive (1)
2. Wait 20ms for any transient effects to settle
3. Begin acquiring data (2) at 6000 samples per second, for 80ms
4. Trigger the stimulus (3) one or more times during the period
5. Switch off current (4) by setting both current strobes set to low
6. Update impedance record
7. Set current strobe B high to switch on current generator; polarity reversed (5)
8-11. As 2-5 above
12. Subtract data from impedance record.

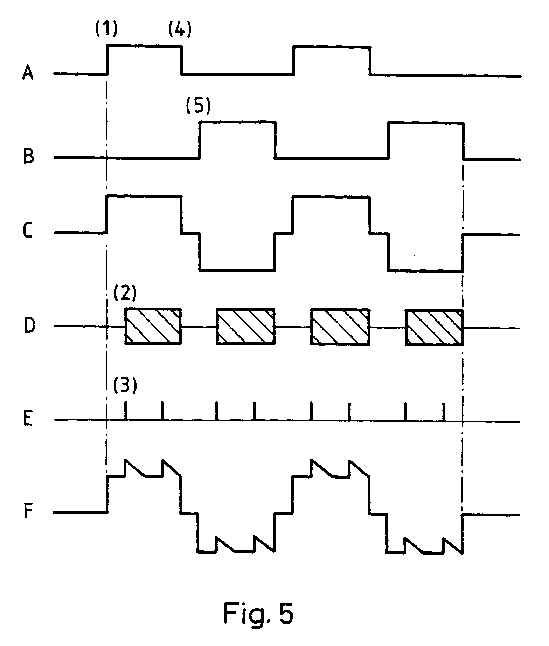

Subsequent pairs of cycles are identical, and a sequence of successive data acquisition cycles is illustrated schematically in Figure 5. The traces shown are as follows:
A. Current strobe A
B. Current strobe B
C. Excitation current
D. Data collection periods
E. Stimulus to nerve
F. Voltage recording

Points (1) to (5) in the described data acquisition cycles are shown in Figure 5.

The above described system can be applied to producing images of activity in the brain with a temporal resolution of the order of the action potential (namely milliseconds or) or less (down to a few microseconds). Ideally, such a technique would be used in humans with a ring of scalp electrodes. It also finds application in humans undergoing neurosurgery (eg. for epilepsy) or in experimental animals if subdural or intracerebral electrodes are needed to provide adequate sensitivity. As the particular technique of the invention in applying interrogatory signals to a body may have advantages in reducing noise effects in EIT techniques in general, the invention may also find application in areas other than neurological investigation.

## Claims

1. A method for use in imaging a body by means of the technique of EIT comprising the steps of:
providing a plurality of electrodes in electrical contact with the body around the periphery of the body;
applying a first electrical input signal to at least one of the electrodes over a first time period;
applying a second electrical input signal to the at least one of the electrodes over a subsequent, second time period, the second electrical input signal being an inverted form of the first electrical input signal;
measuring the resulting electrical output signal at one or more of the remaining electrodes over the first and second time periods; and
calculating the difference between the measured signal obtained during the first time period and that obtained during the second time period, to provide a difference signal.

2. A method according to Claim 1, wherein the first and second electrical input signals are unidirectional signals of equal amplitude and respectively opposite sign.

3. A method according to Claim 1 or Claim 2, wherein the applications of the first electrical input signal and that of the second electrical input signal are continuously alternated, the first and second time periods being equal, and the electrical signal measurements are taken at regular intervals during each time period.

4. A method according to any preceding claim, wherein the measurement step is carried out on a number of different pluralities of electrodes, and images representative of the body are generated using said difference signal.

5. A method according to any preceding claim, wherein the signal application step is carried out in synchronisation with the application of a separate stimulus signal to the body.

6. A method according to any preceding claim, wherein the first and second time period are each between 0.1 and 1.0 seconds.

7. Apparatus for use in imaging a body by means of the technique of EIT comprising:
a plurality of electrodes (E₁-E₁₆) adapted for electrical contact with the body around the periphery of the body;
means (50) for applying a first electrical input signal to at least one of the electrodes over a first time period (T₁);
means (50) for providing an inverted form of the first electrical input signal to produce a second electrical input signal;
means (50) for applying the second electrical input signal to the at least one of the electrodes over a second, subsequent time period (T₂);
means (20,30,40,60) for measuring the resulting electrical output signals at one or more of the remaining electrodes over the first and second time periods; and
means (60) for calculating the difference between the measured signal obtained during the first time period and that obtained during the second time period, to provide a difference signal.

8. Apparatus according to Claim 7, wherein the means for providing the inverted form of the first electrical input signal comprises a switching means controlling an electrical current generator, electrically isolated from the measuring means.

9. Apparatus according to Claim 8, comprising one or more optical isolators.

## Patentansprüche

1. Verfahren zur Verwendung bei der Abbildung eines Körpers mittels der EIT-Technik umfassend die Schritte:
- Bereitstellen einer Mehrzahl von Elektroden in elektrischem Kontakt mit dem Körper um die Peripherie des Körpers,
- Anlegen eines ersten elektrischen Eingangssignals an zumindest eine der Elektroden über eine erste Zeitspanne,
- Anlegen eines zweiten elektrischen Eingangssignals an zumindest eine der Elektroden über eine anschließende zweite Zeitspanne, wobei das zweite elektrische Eingangssignal eine invertierte Form des ersten elektrischen Eingangssignals ist,
- Messen des sich ergebenden elektrischen Ausgangssignals an einer oder mehreren der restlichen Elektroden über die ersten und zweiten Zeitspannen, und
- Berechnen der Differenz zwischen dem gemessenen, während der ersten Zeitspanne erhaltenen Signal und dem während der zweiten Zeitspanne erhaltenen Signal, um ein Differenzsignal bereitzustellen.

2. Verfahren nach Anspruch 1, bei dem die ersten und zweiten elektrischen Eingangssignale unidirektionale Signale mit gleicher Amplitude und entsprechend gegensätzlichen Vorzeichen sind.

3. Verfahren nach Anspruch 1 oder 2, bei dem die Anwendungen des ersten elektrischen Eingangssignals und des zweiten elektrischen Eingangssignals beständig verändert werden, wobei die ersten und zweiten Zeitspannen gleich sind und die elektrischen Signalmessungen in regelmäßigen Abständen während jeder Zeitspanne durchgeführt werden.

4. Verfahren nach einem der vorhergehenden Ansprüche, bei dem der Meßschritt an mehreren unterschiedlichen Elektrodensätzen durchgeführt wird, wobei den Körper repräsentierende Bilder unter Verwendung des Differenzsignals erzeugt werden.

5. Verfahren nach einem der vorhergehenden Ansprüche, bei dem der Signalanwendungsschritt in Synchronisation mit der Anwendung eines separaten Reizsignals auf den Körper durchgeführt wird.

6. Verfahren nach einem der vorhergehenden Ansprüche, bei dem die ersten und zweiten Zeitspannen zwischen 0,1 und 1,0 Sekunden liegen.

7. Vorrichtung zur Verwendung bei der Abbildung eines Körpers mittels der EIT-Technik umfassend:
- eine Mehrzahl von Elektroden (E₁-E₁₆), die zum elektrischen Kontakt mit dem Körper um die Peripherie des Körpers ausgelegt sind,
- Einrichtungen zum Anlegen eines ersten elektrischen Eingangssignals an zumindest eine der Elektroden über eine erste Zeitspanne,
- Einrichtungen zum Bereitstellen einer invertierten Form des ersten elektrischen Eingangssignals zur Erzeugung eines zweiten elektrischen Eingangssignals,
- Einrichtungen zum Anlegen des zweiten elektrischen Eingangssignals an zumindest eine der Elektroden über eine zweite, darauffolgende Zeitspanne,
- Einrichtungen zum Messen des sich ergebenden elektrischen Ausgangssignals an einer oder mehreren der restlichen Elektroden über die ersten und zweiten Zeitspannen, und
- Einrichtungen zum Berechnen der Differenz zwischen dem gemessenen, während der ersten Zeitspanne erhaltenen Signal und dem während der zweiten Zeitspanne erhaltenen Signal, um ein Differenzsignal bereitzustellen.

8. Vorrichtung nach Anspruch 7, wobei die Einrichtung zum Bereitstellen der invertierten Form des ersten elektrischen Eingangssignals eine Schalteinrichtung umfaßt, die einen elektrischen Stromgenerator steuert, der elektrisch von der Meßeinrichtung isoliert ist.

9. Vorrichtung nach Anspruch 8, die einen oder mehrere optische Trennschalter umfaßt.

## Revendications

1. Procédé destiné à être utilisé pour la réalisation d'images d'un corps au moyen de la technique de tomographie à impédance électrique, comprenant les étapes consistant à :
disposer une pluralité d'électrodes en contact électrique avec le corps autour de la périphérie du corps ;
appliquer un premier signal d'entrée électrique à au moins l'une des électrodes au cours d'une première période de temps ;
appliquer un deuxième signal d'entrée électrique à l'électrode au nombre d'au moins une au cours d'une deuxième période de temps ultérieure, le deuxième signal d'entrée électrique étant une forme inversée du premier signal d'entrée électrique ;
mesurer le signal de sortie électrique résultant sur une ou plusieurs des électrodes restantes au cours des première et deuxième périodes de temps ; et
calculer la différence entre le signal mesuré obtenu au cours de la première période de temps et celui obtenu au cours de la deuxième période de temps, de façon à délivrer un signal de différence.

2. Procédé selon la revendication 1, dans lequel les premier et deuxième signaux d'entrée électriques sont des signaux unidirectionnels d'amplitude égale et de signe respectivement opposé.

3. Procédé selon la revendication 1 ou la revendication 2, dans lequel les applications du premier signal d'entrée électrique et celles du deuxième signal d'entrée électrique alternent de façon continue, les première et deuxième périodes de temps étant égales, et les mesures de signaux électriques étant effectuées à intervalles réguliers au cours de chaque période de temps.

4. Procédé selon l'une quelconque des revendications précédentes, dans lequel l'étape de mesure est effectuée sur un certain nombre de pluralités différentes d'électrodes, et des images représentatives du corps sont générées en utilisant ledit signal de différence.

5. Procédé selon l'une quelconque des revendications précédentes, dans lequel l'étape d'application de signal est effectuée en synchronisme avec l'application d'un signal de stimulus séparé au corps.

6. Procédé selon l'une quelconque des revendications précédentes, dans lequel les première et deuxième périodes de temps sont chacune comprises entre 0,1 et 1,0 secondes.

7. Dispositif destiné à être utilisé à la réalisation d'images d'un corps au moyen de la technique de tomographie à impédance électrique, comprenant :
une pluralité d'électrodes (E₁ - E₁₆) adaptées pour venir en contact électrique avec le corps autour de la périphérie du corps ;
des moyens pour appliquer un premier signal d'entrée électrique à au moins l'une des électrodes au cours d'une première période de temps ;
des moyens pour délivrer une forme inversée du premier signal d'entrée électrique afin de produire un deuxième signal d'entrée électrique ;
des moyens pour appliquer le deuxième signal d'entrée électrique à l'électrode au nombre d'au moins une au cours d'une deuxième période de temps ultérieure ;
des moyens pour mesurer les signaux de sortie électriques résultants sur une ou plusieurs des électrodes restantes au cours des première et deuxième périodes de temps ; et
des moyens pour calculer la différence entre le signal mesuré obtenu au cours de la première période de temps et celui obtenu au cours de la deuxième période de temps, de façon à délivrer un signal de différence.

8. Dispositif selon la revendication 7, dans lequel les moyens pour délivrer la forme inversée du premier signal d'entrée électrique comprennent des moyens de commutation commandant un générateur de courant électrique, électriquement isolé des moyens de mesure.

9. Dispositif selon la revendication 8, comprenant un ou plusieurs isolateurs optiques.
